# EUROPEAN PATENT SPECIFICATION

(11) **EP 2 280 677 B1**
(45) Date of publication and mention of the grant of the patent: **27.07.2016**
(21) Application number: 09727264.5
(22) Date of filing: 01.04.2009
(51) Int. Cl.: A61F 9/008, A61F 2/14

(54) **CORNEAL IMPLANT SYSTEM AND INTERFACE**
HORNHAUTIMPLANTATSYSTEM UND SCHNITTSTELLE
SYSTÈME ET INTERFACE D'IMPLANT DE LA CORNÉE

(30) Priority: 01.04.2008 US 41544 P
(43) Date of publication of application: 09.02.2011
(73) Proprietor: AMO Development, LLC, Santa Ana, CA 92705 (US); Catlin, Scott J., Orange, CA 92867 (US); Tuan, Kuang-Mon Ashley, Mountain View, CA 94040 (US); Pang, Andrew, Cost Mesa, CA 92626 (US)
(72) Inventor: CATLIN, Scott, J., Orange, CA 92867 (US); TUAN, Kuang-mon, Ashley, Mountain View, CA 94040 (US); PANG, Andrew, Cost Mesa, CA 92626 (US)
(74) Representative: Hoffmann Eitle
(86) International application number: PCT/US2009/039200
(87) International publication number: WO 2009/124143

(56) References cited:
- WO-A-03/005920
- WO-A-03/022168
- WO-A-03/081379
- WO-A-2008/030699
- WO-A-2008/131888
- US-A1- 2007 161 972

## Description

### BACKGROUND OF THE INVENTION

### Field of the Invention

The field of the present invention is generally related to corneal implants and more particularly, to corneal implant systems, interfaces, and methods.

### Background

Intracorneal implants have been developed in an attempt to correct abnormalities of the eye. The intracorneal implant may be implanted or inserted into a desired region of the cornea, such as within the stroma of the cornea. In one technique, a stromal pocket is created by making an incision in the stromal layer of the cornea or by removing a small amount of stromal tissue during the implant procedure. The implant is typically placed in the stromal pocket to reshape the cornea, alter refractive properties of the cornea, or both.

In the past, stromal pockets have been prepared to accommodate implants by mechanically incising and separating corneal lamellae. For example, stromal pockets may be manually prepared to accommodate lens and disk-shaped corneal implants. More recently, surgical lasers using pulsed laser beams have been used to incise corneal tissue for a variety of ophthalmic procedures, such as to produce corneal flaps. Typically, the surgical laser is programmed to perform one or more specific incisions to scan a stromal bed circumscribed by an edge to create a corneal flap.

Many different types of corneal implants are currently available or in development. For example, corneal rings, such as a single continuous annular ring, two half-ring components, a ring segment, a pair of crescent-shaped corneal implants, or a variety of other shaped implants, either single or multi-component types, can also be embedded within the stromal tissue (e.g., surrounding a central optical zone of the cornea). Development continues for additional implants having different shapes and configurations.

Accordingly, it is desirable to provide systems and methods for ophthalmic laser surgery that operate with and manage multiple corneal implant types. It is also desirable to provide systems and methods for establishing a corneal pocket or other retaining region suitable for accommodating a selected corneal implant. It is also desirable to provide systems and methods for modifying and customizing the characteristics of the corneal pocket or other retaining region once identified for the selected corneal implant. It is further desirable to provide systems and methods for corneal laser surgery that automatically selects a pre-programmed incision procedure based on a corneal implant desired for implantation. Additionally, other desirable features and characteristics of the present invention will become apparent from the subsequent detailed description and the appended claims, taken in conjunction with the accompanying drawings and the foregoing technical field and background.

WO 03/005920 A1 relates to a surgical method for vision correction in which a laser beam is focused to a pre-selected start point within the stromal tissue. According to a pre-planned procedure, the focal point is located on the posterior surface of the prescribed stromal pocket.

### SUMMARY OF THE INVENTION

The present invention is as defined in the independent claims and is directed towards systems and graphical user interfaces. In one embodiment, a system is provided including a display operable to present a plurality of images representing a plurality of corneal implants, a laser assembly operable to output a pulsed laser beam, and a control unit coupled to the laser assembly and the display. The control unit is configured to direct the laser assembly in response to a selected image of the plurality of images to produce a cavity in the cornea via the pulsed laser beam. The cavity is configured to receive a first corneal implant of the plurality of corneal implants corresponding to the selected image.

In another embodiment, a graphical user interface renderable by a display device within a graphical window is provided. The interface includes a first area to facilitate a specification of a corneal implant from a plurality of corneal implants, and a second area to facilitate a specification of one or more incision properties. The second area is modified in response to the specification of the corneal implant.

In another embodiment, a system is provided for incising a cornea. The system includes a read unit operable to determine an identification representing a corneal implant, a laser assembly operable to output a pulsed laser beam, and a control unit coupled to the read unit and the laser assembly. The control unit is configured to direct the laser assembly to form a cavity in the cornea via the pulsed laser beam based on the identification. The cavity is configured to receive the corneal implant.

### BRIEF DESCRIPTION OF THE DRAWINGS

In the drawings, wherein like reference numerals refer to similar components:
FIG. 1 is a block diagram of an ophthalmic laser system in accordance with one embodiment of the present invention;
FIG. 2 is a schematic diagram of an ophthalmic laser system in accordance with another embodiment;
FIG. 3 is a block diagram of an ophthalmic laser system in accordance with another embodiment;
FIG. 4 is an elevational view of a display screen illustrating a graphical user interface in accordance with one embodiment;
FIG. 5 is an elevational view of a display screen illustrating a graphical user interface in accordance with another embodiment;
FIG. 6 is an elevational view of a display screen illustrating a graphical user interface in accordance with another embodiment;
FIG. 7 is a flow diagram of a method for ophthalmic surgery given as an example.

### DETAILED DESCRIPTION

The present invention provides systems and graphical user interfaces for ophthalmic surgery. More particularly, the present invention provides systems and graphical user interfaces for ophthalmic surgery with corneal implants (e.g., inlays, onlays, and the like). In one embodiment, the system determines or detects the type of corneal implant desired to be implanted, selects a pre-programmed procedure from among a group of updateable pre-programmed procedures, and directs a laser assembly to produce a cavity in the cornea using a pulsed laser beam. The selected pre-programmed procedure corresponds with the desired corneal implant, and the resulting cavity corresponds with the desired corneal implant (e.g., conforms to the shape, material properties, and/or the like, associated with the desired corneal implant). A user may optionally modify the pre-programmed procedure to customize the resulting cavity.

In general, photoalteration of a material may be accomplished using a pulsed laser beam that is directed (e.g., via a scanner) at a desired region of the material. For example, a pulsed laser beam may be controlled to scan the desired region (e.g., on the surface or sub-surface) and to create a separation of the material (e.g., which may be used to produce a flap of the material, to separate a portion of the material for transplants, or for a variety of other uses). Typically, a pulsed laser beam is focused onto a desired area of the material to photoalter the material in this area and, in some instances, the associated peripheral area. Examples of photoalteration of the material include, but are not necessarily limited to, chemical and physical alterations, chemical and physical breakdown, disintegration, ablation, vaporization, or the like. Although various embodiments of systems, graphical user interfaces, and methods for ophthalmic surgery are described, these systems, graphical user interfaces, and methods may be applied to other biological tissues and other materials.

One example of photoalteration using pulsed laser beams is the photodisruption (e.g., via laser induced optical breakdown) of a material. Localized photodisruptions can be placed at or below the surface of the material to produce high-precision material processing. For example, a micro-optics scanning system may be used to scan the pulsed laser beams to produce an incision in the material and create a flap, a pocket, or a cavity therefrom. The term "scan" or "scanning" refers to the movement of the focal point of the pulsed laser beam along a desired path (e.g., along an x-axis, a y-axis, a z-axis, or any combination thereof) or in a desired pattern.

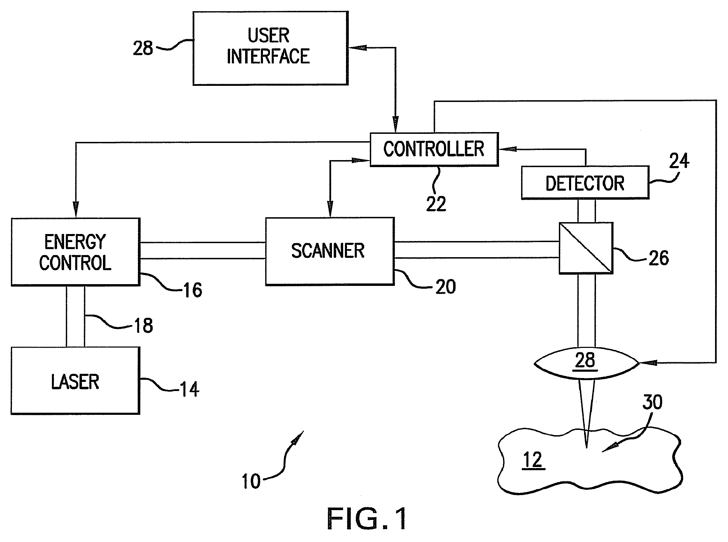

Referring to the drawings, an ophthalmic laser system 10 is shown in FIG. 1. The system 10 includes, but is not necessarily limited to, a laser source 14 capable of generating a pulsed laser beam 18, an energy control module 16 for varying the pulse energy of the pulsed laser beam 18, a scanner 20, a controller 22, a user interface 28, and focusing optics 28 that direct one or more focal points 30 of the pulsed laser beam 18 onto the surface of or within the eye 12 (e.g., sub-surface into the corneal region, such as sub-epithelium, sub-Bowman's layer, within the stroma, substantially adjacent to Descemet's membrane, and the like). The controller 22 (e.g., a processor operating suitable control software) communicates with one or more of the scanner 20, user interface 28, and focusing optics 28 to control the direction of the focal point 30 as it is scanned along the desired region of the eye. The user interface 28 provides the user with a variety of input parameters for controlling the system 10. For example, the pulsed laser beam 18 may be controlled based on one or more input parameters to produce a desired incision. In one embodiment, the user interface 28 determines the type of corneal implant to be used for a particular ophthalmic procedure. Based on this determination, the controller 22 automatically configures the system 10 to a set of pre-determined operating values corresponding with the specific corneal implant. For customization (e.g., patient specific factors, historical performance, and the like), the user may modify one or more of these operating values via the user interface 28.

In one embodiment, software, firmware, or the like, can be used (executed by the controller 22 to control or direct one or more components of the system 10) to command the actions and placement of the scanner via a motion control system, such as a closed-loop proportional integral derivative (PID) control system. In this embodiment, the system 10 further includes a beam splitter 26 and a detector 24 coupled to the controller 22 to provide a feedback control mechanism for the pulsed laser beam 18. The beam splitter 26 and detector 24 may also be omitted in other embodiments, for example, with different control mechanisms.

Movement of the focal point 30 of the pulsed laser beam 18 is accomplished via the scanner 20 in response to the controller 22. In one embodiment, the scanner 20 scans the pulsed laser beam 18 to produce an incision in the desired region of the cornea. To provide the pulsed laser beam 18, a chirped pulse laser amplification system, such as described in U.S. Pat. No. RE37,585, may be used for photoalteration. U.S. Pat. Publication No. 2004/0243111 also describes other methods of photoalteration. Other devices or systems may be used to generate pulsed laser beams. For example, non-ultraviolet (UV), ultrashort pulsed laser technology can produce pulsed laser beams having pulse durations measured in femtoseconds. Some of the non-UV, ultrashort pulsed laser technology may be used in ophthalmic applications. For example, U.S. Pat. No. 5,993,438 discloses a device for performing ophthalmic surgical procedures to effect high-accuracy corrections of optical aberrations. U.S. Pat. No. 5,993,438 discloses an intrastromal photodisruption technique for reshaping the cornea using a non-UV, ultrashort (e.g., femtosecond pulse duration), pulsed laser beam that propagates through corneal tissue and is focused at a point below the surface of the cornea to photodisrupt stromal tissue at the focal point.

In this case, the focusing optics 28 direct the pulsed laser beam 18 toward a region 12 of the eye (e.g., onto the cornea) for plasma mediated (e.g., non-UV) photoablation of superficial tissue on the eye, or into the stroma for intrastromal photodisruption of tissue. The system 10 is capable of generating the pulsed laser beam 18 with physical characteristics similar to those of the laser beams generated by a laser system disclosed in U.S. Pat. Nos. 4,764,930 and 5,993,438, or the like. For example, the system 10 can produce a non-UV, ultrashort pulsed laser beam for use as an incising laser beam. This pulsed laser beam preferably has laser pulses with durations as long as a few nanoseconds or as short as a few femtoseconds. For intrastromal photodisruption of the tissue, the pulsed laser beam 18 has a wavelength that permits the pulsed laser beam 18 to pass through the cornea without absorption by the corneal tissue. The wavelength of the pulsed laser beam 18 is generally in the range of about 3 µm to about 1.9 nm, and preferably between about 400 nm to about 3000 nm. For accomplishing photodisruption of stromal tissues at the focal point, the irradiance of the pulsed laser beam 18 is preferably greater than the threshold for optical breakdown of the tissue. Although a non-UV, ultrashort pulsed laser beam is described in this embodiment, the pulsed laser beam 18 may have other pulse durations and different wavelengths in other embodiments (e.g., UV wavelengths for excimer applications and the like).

Scanning is accomplished with the scanner 20 via the controller 22 by selectively moving the focal point(s) 30 to produce a structured scan pattern (e.g., a raster pattern, a spiral pattern, or the like) of scan spots. Operating the scanner 20 to scan this structured pattern is particularly useful for controlling the spacing between scan spots of the pattern. The step rate at which the focal point 30 is moved is referred to herein as the scan rate. For example, the scanner 20 can operate at scan rates between about 10 kHz and about 400 kHz, or at any other desired scan rate. In one embodiment, the scanner 20 generally moves the focal point of the pulsed laser beam 18 through the desired scan pattern at a substantially constant scan rate while maintaining a substantially constant separation between adjacent focal points. Further details of laser scanners are known in the art, such as described, for example, in U.S. Patent No. 5,549,632.

In one embodiment, the scanner 20 includes, but is not necessarily limited to, a pair of scanning mirrors or other optics (not shown) to angularly deflect and scan input beam (e.g, the pulsed laser beam 18). For example, scanning mirrors driven by galvanometers may be employed where each of the mirrors scans along different orthogonal axes (e.g., an x-axis and a y-axis). A focusing objective (not shown), having one or more lenses, images the input beam onto a focal plane of the system 10. The focal point 30 may thus be scanned in two dimensions (e.g., along the x-axis and the y-axis) within the focal plane of the system 10. Scanning along the third dimension, i.e., moving the focal plane along an optical axis (e.g., a z-axis), may be achieved by moving the focusing objective, or one or more lenses within the focusing objective, along the optical axis.

For ophthalmic applications (e.g., preparing a cornea for flap separation, corneal transplant, preparing a pocket or cavity in the cornea, or the like), an area (e.g., substantially circular, oval, or other shape) may be scanned with a scan pattern based on the movement of the scanning mirrors. As the focal point 30 is scanned along a corneal bed, the pulsed laser beam 18 photoalters the stromal tissue. Using structured patterns, the distribution of scan spots is generally determined by the pulse frequency, the scan rate, and the amount of scan line separation. Generally, higher scan rates, enable shorter procedure times by increasing the rate at which corneal tissue can be photoaltered. For example, the scan rates may be selected from a range between about 30 MHz and about 1 GHz with a pulse width in a range between about 300 picoseconds and about 10 femtoseconds, although other scan rates and pulse widths may be used.

The system 10 may additionally acquire detailed information about optical aberrations to be corrected, at least in part, using the system 10. Examples of such detailed information include, but are not necessarily limited to, the extent of the desired correction, and the location in the cornea of the eye associated with the correction (e.g., where the correction can be made most effectively). The refractive power of the cornea may be used to indicate corrections. Wavefront analysis techniques, made possible by devices such as a Hartmann-Shack type sensor (not shown), can be used to generate maps of corneal refractive power. Other wavefront analysis techniques and sensors may also be used (e.g., based on phase diversity or other techniques). The maps of corneal refractive power, or similar refractive power information provided by other means, such as corneal topographs or the like, can then be used to identify and locate the optical aberrations of the cornea that require correction. Optical aberration information acquired by other methods may also be utilized with the system 10, such as imaging techniques using optical coherence tomography (OCT) or other interferometric imaging schemes.

In general, when the laser source 14 is activated, the focal spot 30 is selectively directed (e.g., via the scanner 20) along a beam path to photoalter stromal tissue. For example, the focal spot 30 is moved along a predetermined length of the beam path in one reference area. The pulsed laser beam 18 is then redirected through another reference area, and the process of photoalteration is repeated. The sequence for directing the pulsed laser beam 18 through individually selected reference areas can be varied, and the extent of stromal tissue photoalteration while the incising laser beam is so directed, can be varied. Specifically, as indicated above, the amount of photoalteration can be based on the refractive power map. On the other hand, the sequence of reference areas that is followed during a customized procedure will depend on the particular objectives of the procedure.

The scanner 20 may also scan a predetermined pattern using one or more scan patterns to one or more combinations of these reference areas or scan a single line (e.g., to produce a sidecut or continuous incision). One example of an ophthalmic scanning application is the creation of a cavity or a pocket in the cornea. The laser focal spot 30 of the pulsed laser beam 18 may be scanned at a pre-determined subsurface depth of the cornea (e.g., such as sub-epithelium, sub-Bowman's layer, within the stroma, substantially adjacent to Descemet's membrane, and the like) to form a pocket bed. An incision may be made to connect the surface of the eye with the pocket bed. The desired corneal implant, or specific component of a multi-component corneal implant, is then inserted through this incision. Although a single incision is described in this embodiment, additional incisions may be made to connect the surface of the eye with the pocket bed. In another embodiment, intrastromal tissue is photoaltered by the system 10 so as to create an isolated lenticle of intrastromal tissue, and the lenticle of tissue can then be removed from the cornea (e.g., through the incision) to create the pocket. The incision selection may be based at least in part on the alteration of the shape of the cornea as particular to the vision correction procedure.

Another example of an ophthalmic scanning application is a laser assisted in-situ keratomilieusis (LASIK) type procedure where a flap is cut from the cornea to establish extracorporeal access to the tissue that is to be photoaltered. The flap may be created using random scanning or one or more scan patterns of pulsed laser beams. To create the corneal flap, a sidecut is created around a desired perimeter of the flap such that the ends of the sidecut terminate, without intersection, to leave an uncut segment. This uncut segment serves as a hinge for the flap. The flap is separated from the underlying stromal tissue by scanning the laser focal point across a resection bed, the perimeter of which is approximately defined by and slightly greater than the sidecut. Once this access has been achieved, photoalteration is completed and the residual fragments of the photoaltered tissue are removed from the cornea. In another embodiment, the sidecut may be created completely around a desired perimeter (e.g., with the ends terminating with one another) to separate a portion of corneal tissue (e.g., for corneal transplant or the like) from the cornea. With the availability of these different ophthalmic scanning applications in the system 10, ophthalmic procedures may be combined to complement the optical benefits provided by the desired corneal implant with the refractive corrections provided by LASIK, corneal transplants, or a variety of other ophthalmic procedures. For example, a corneal flap may be formed followed by photoalteration of the flap bed (e.g., creation of a custom-shaped regions) to receive the inlay or implant.

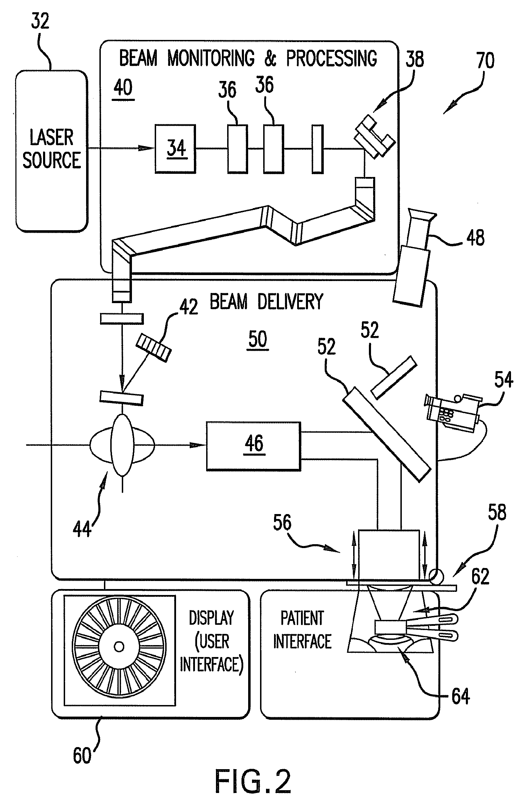

FIG. 2 is a block diagram of an ophthalmic laser system 70 in accordance with another embodiment of the present invention. The ophthalmic laser system 70 illustrates the optical path of the pulsed laser beam in greater detail and includes, but is not necessarily limited to, a laser source 32 providing a pulsed laser beam (e.g. from the laser 14 shown in FIG. 1), a beam monitoring and processing module 40, a beam delivery module 50, and a user interface 60. The pulsed laser beam is supplied to the beam monitoring and processing module 40 where operating characteristics of the pulsed laser beam are controlled, such as the pulse energy (e.g., varied by the energy control module 16 shown in FIG. 1), a focal point separation, a minimum sub-surface depth of the pulsed laser beam, and the like). The beam delivery module 50 scans the pulsed laser beam along a desired scan region. In this embodiment, the ophthalmic laser system 70 can be coupled to an eye 64 via a patient interface 62, and the patient interface 62 may be coupled to the ophthalmic laser system 70 at a loading deck 58, for example. A display is provided by the user interface 60 for viewing the eye 64 undergoing laser treatment.

In one embodiment, the beam monitoring and processing module 40 includes, but is not necessarily limited to, an energy attenuator 34, one or more energy monitors 36, and an active beam positioning mirror 38. The pulsed laser beam is directed from the laser source 32 to the energy attenuator 34, then to the energy monitor 36, and then to the active beam positioning mirror 38. The active beam positioning mirror 38 directs the pulsed laser beam from the beam monitoring and processing module 40 to the beam delivery module 50. Using the energy attenuator 34 and energy monitor 36, the pulse energy of the pulsed laser beam may be varied to desired values. Additionally, the spatial separation of the focal points of the pulsed laser beam may be varied by the beam monitoring and processing module 40.

The beam delivery module 50 scans the pulsed laser beam at the desired scan region (e.g., a sub-surface region of the eye 64, such as within the corneal epithelium and on or within Bowman's layer, the stroma, Descemet's membrane, the endothelium, and the like). In one embodiment, the beam delivery module 50 includes, but is not necessarily limited to, a beam position monitor 42, an x-y scanner 44 (e.g., such as the scanner 20 shown in FIG. 1), a beam expander 46, one or more beam splitters 52, and a z-scanning objective 56. In this embodiment, the beam delivery module 50 additionally includes an operating microscope 48 and a video camera 54 to enhance viewing of the eye 64.

The pulsed laser beam is received from the beam monitoring and processing module 40 by the x-y scanner 44 and directed to the beam expander 46, and the beam expander 46 directs the pulsed laser beam to the z-scanning objective via the beam splitter(s) 52. The z-scanning objective 56 can vary the focal point depth of the pulsed laser beam. For example, the z-scanning objective 56 can vary the focal point depth to the minimum focal point depth determined for the eye 64 or the desired region of the eye 64 and for the selected operating pulse energy and operating focal point separation.

The configuration of the ophthalmic laser system 70 may vary as well as the organization of the various components and sub-components of the ophthalmic laser system 70. For example, some sub-components of the beam delivery module 50 may be incorporated with the beam monitoring and processing module 40 and vice versa.

For corneal implant applications, the user interface 28 presents a variety of corneal implant options for selection by the user. In one embodiment, the user interface 28 includes, but is not necessarily limited to, a display and an input device, such as a touch-screen, a mouse, a keyboard, a touch-pad, or the like. Multiple images representing different types of corneal implants are presented on the display, and the user may select the appropriate corneal implant for a particular ophthalmic procedure. For example, the user may indicate a corneal implant selection by touching the image on the touch-screen corresponding with the appropriate corneal implant. A variety of pre-programmed and updateable incisions are available to the system 10, 70 for forming an appropriate cavity in the cornea to receive the selected corneal implant. For example, incisions associated with IntraLase^{®} enabled keratoplasty (IEK) and other lamellar keratoplasty or penetrating keratoplasty procedures may be incorporated as part of a suite of pre-programmed incisions utilized by the system 10, 70.

In another embodiment, the user interface 28 includes, but is not necessarily limited to, a read device, such as a radio frequency identification (RFID) interrogator, a bar code scanner, and the like. In this embodiment, the user simply positions the actual corneal implant label or packaging for identification by the read device. For example, the corneal implant may be packaged with an RFID tag. With an RFID interrogator, the identification may be made by positioning the packaging within proximity of the RFID interrogator. In another example, the corneal implant may be packaged with a bar code on the exterior of the packaging. With a bar code scanner, the identification may be made by positioning the bar code within scanning range of the bar code scanner.

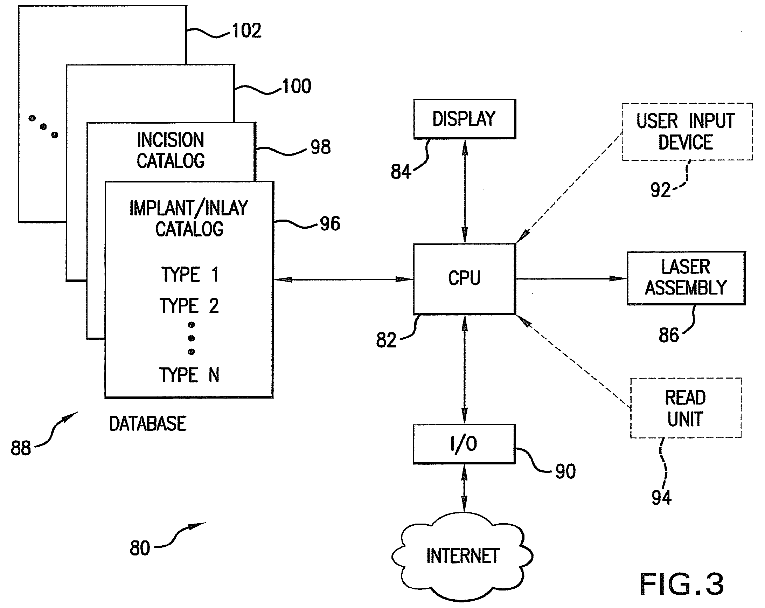

Once desired corneal implant is identified by the user interface 28, this identification is provided to the controller 22 (FIG. 1). The controller 22 selects a pre-determined procedure, corresponding with the identified corneal implant, from among a stored database of pre-determined procedures. Referring to FIG. 3, an ophthalmic laser system 80 is shown illustrating corneal implant related communication or data flow in accordance with one embodiment. The ophthalmic laser system 80 includes, but is not necessarily limited to, a central processing unit (CPU) 82, a display 84 coupled to the CPU 82, a laser assembly 86 coupled to the CPU 82, a memory 88 having a database 96, 98, 100, 102 coupled to the CPU 82, and an input/output (IO) device 90 coupled to the CPU 82. The display 84 may have a touch-sensitive screen, as previously mentioned regarding the user interface 28 shown in FIG. 1. Additionally, at least one of a user input device 92 (e.g., a mouse, a keyboard, a touch-pad, and the like) and a read unit 94 (e.g., RFID interrogator, bar code scanner, and the like) may be coupled to the CPU 82 for different embodiments of the ophthalmic laser system 80. For example, as previously mentioned, the user input device 92 facilitates user selection of the desired corneal implant type, and the read unit 94 facilitates identification of the desired corneal implant type.

In one embodiment, the memory 88 also stores an operating system module and a graphical user interface (GUI) module. The operating system module may include executable instructions for handling various system services, such as file services or for performing hardware dependant tasks. The GUI module may rely upon standard techniques to produce graphical components on the display 84, e.g., windows, icons, buttons, menus, drop-down menus, and the like, examples of which are discussed below. The GUI module may include executable instructions to receive input from a pointer device (e.g., a mouse, a keyboard, a touch-pad, and the like) or touch-screen and display a cursor on the display 84. Additionally, the GUI module includes executable instructions for selecting items in the GUI. The executable modules stored in memory 88 are exemplary. It should be appreciated that the functions of the modules may be combined or divided into additional modules.

The identification of the desired corneal implant type is determined by user selection on the touch-sensitive screen of the display 84, determined by user input via the user input device 92, or detected by the read unit 94 and communicated to the CPU 82. With the identification, the CPU 82 retrieves a pre-determined incision procedure from the database 96, 98, 100, 102. In one embodiment, the database includes a catalog of corneal implant types 96 and a catalog of pre-determined incision procedures 98. Although described with catalogs, the database may be implemented with one or more look-up tables or other data management configurations. Each of the incision procedures corresponds to a different corneal implant type, and the catalogs 96 and 98 may be periodically updated via the IO device 90. In one embodiment, the IO device 90 is a media-specific reader or multimedia reader compatible with a variety of memory storage devices (e.g., flash memory, portable disk drive, floppy disk, memory stick, compact disc, and the like). In the alternative or supplementary thereto, the IO device 90 may have network connectivity, such as to another network capable device (e.g., laptop computer, desktop computer, notebook computer, portable computer, and the like) or the Internet, via a standard network connection or wireless connection. In all embodiments, additional corneal implant types and corresponding incision procedures are accessible by the IO device 90. For example, new corneal implant types and the corresponding incision procedures may be down-loaded via the media reader or retrieved from the Internet and entered into the catalogs 96 and 98, respectively. Obsolete corneal implant types, and the corresponding incision procedures, may also be removed from the catalogs 96 and 98 based on the updated information. Additionally, the catalogs 96, 98 may be customizable (e.g., in general, for all selections associated with a type of procedure and/or individually by patient).

The catalog of corneal implant types 96 includes a variety of implant types (e.g., a Type 1 implant, a Type 2 implant, ..., and a Type N implant). The incision procedures in the catalog of incision procedures 98 may be similarly designated for association with the corresponding corneal implant type, although a particular incision procedure may be applicable to more than one of the corneal implant types. Some examples of corneal implant types include, but are not necessarily limited to, a variety of discs with varying radii of curvatures associated with the anterior and/or posterior surfaces of the disc and/or with varying cross-sectional shapes, single or multi-perforated discs, multi-component implants, crescents, rings, lenses, and the like. Additional corneal implant types may be derived from variants of these examples based on pre-determined sizes or dimensions. Combinations of corneal implant types and incisions to provide refractive corrections may also be cataloged. In one embodiment, the corneal implant types are presented on the display 84 as user-selectable representations (e.g., names, images, icons, buttons, and the like).

Depending on the identification of the corneal implant type, the CPU 82 retrieves the corresponding incision procedure from the catalog of incision procedures 98. Each corneal implant type is associated with specific physical characteristics or properties, such as dimensions (e.g., width, height, thickness, shape, profile, diameter, and the like), material of construction, elasticity, hardness, material strength, and the like, at least some of which are used to pre-determine various aspects of the corresponding incision procedure. For example, the dimensions of a corneal implant type may be used to pre-program incision parameters (e.g., length, depth, volume, shape, and the like) to be produced by the incision procedure. The incision procedure is preferably pre-programmed to produce a cavity that conforms to the shape of the desired corneal implant type and permits insertion of the desired corneal implant. For example, the dimensions and characteristics of each corneal implant in the catalog 96 are used to program software that operates the laser assembly 86 to cut a cavity or pocket for receiving the corresponding corneal implant.

In one embodiment, at least some of the incision parameters are presented on the display 84 and may be modified by the user (e.g., for customized ophthalmic procedures). For example, drop-down menus, input blocks, incremental blocks, and the like may be used to set or modify one or more incision parameters (e.g., default incision parameter settings), the number and/or type of incisions, and the like associated with the particular incision procedure. These modifications enhance user customization of the entire ophthalmic procedure by accounting for user experience, historical procedural results, patient specific factors, and the like. For example, customization may be based on wavefront analysis, OCT maps, pupil dynamics (e.g., to assist with centration, incision selection, and/or implant selection), centration, iris registration, and the like.

Refractive corrections (e.g., existing refractive corrections, such as provided by a corneal implant, or as needed at a later time) may change over time. Multiple photoablations of the cornea over a long duration may not be feasible because of the limited amount of corneal tissue and/or the integrity of the cornea. Additionally, the patient's corneal stromal bed may not be sufficient for ablation with a particular refractive procedure. In another embodiment, the refractive characteristics associated with the selected corneal implant may be altered by the laser assembly 86 to fine-tune the overall refractive correction provided by the corneal implant. This may be performed immediately following implant or at a subsequent time. Multiple refractive adjustments may be performed on the patient's corneal implant using the prior knowledge of the corneal implant characteristics or properties. For example, the corneal implant, and associated incisions and cavity, for a particular patient may be stored by the system 10, 70, 80 for subsequent recall and additional refractive correction (e.g., removal of the implant, exchange of the implant, ablation of the implant, and the like, for over or under correction, long-term refractive error correction, provision of monovision for presbyopic relief, and the like).

The corneal implant may be selected to provide a gross refractive correction (e.g., by modifying lens shape, thickness, and/or refractive index differences), and surface corneal implant laser ablation can provide fine-tuning or refractive error and higher order aberrations. Myopic patients retaining the option for future monovision correction to relieve presbyopia symptoms may opt for a partial correction of the myopic prescription and correct the remaining myopia (e.g., from about -0.75D to about -2.50D correction) via a corneal implant. Over time as presbyopia effects increase, the myopic patient can seek to remove the corneal implant and result in a low myopic eye with monovision effect. For example, a thirty-five (35) year old patient with -6.00D for both eyes can have a -6.00D correction via photoalteration of the dominant eye and a -4.00D correction of the non-dominant corneal stroma via photoalteration with a -2.00D correction via a corneal inlay (e.g., lens shape or ablation of the inlay). The inlay may be subsequently removed at a presbyopic year to result in a -2.00 myopia on the non-dominant eye and a 2.00D add monovision.

Post-LASIK patients desiring monovision correction without cornea ablation may select a corneal implant to create a low degree of myopia in one eye to provide near visual relief. For example, a fifty-five (55) year old post-LASIK patient with piano refraction desiring monovision to relieve presbyopic symptoms can have a corneal inlay with +1.50D correction implanted under the LASIK flap on one eye (e.g., the non-dominant eye).

Patients with adult on-set myopia or progressive myopia may increase myopic refractive error over time during adulthood. Full or partial correction may be performed on the cornea during a first photoablation with an intra-corneal lens/corneal inlay procedure used for future fine tuning via photoablation on the intra-corneal lens/corneal inlay. The lenses/inlays may be removed and exchanged with different lenses/inlays (e.g., adjusted lens diameter, thickness curvature, and/or refractive index) to provide better myopic effect.

A hyperopic patient with a cycloplegic refraction significantly greater than the manifest refraction is generally classified as latent hyperopia. Patients with latent hyperopia may not tolerate full refractive correction. A latent hyperope without full correction may experience hyperopic progression as accommodation relaxes or declines with aging. Currently, to correct latent hyperopia, the manifest refraction is corrected or the two refractions are averaged. Further correction may be needed as the patient gets used to the correction and/or the accommodation is relaxed or reduced. Full or partial correction on the cornea may be performed during the first photoablation, and an intra-corneal lens may be implanted for future fine tuning via ablation on the intra-corneal lens. For example, a twenty-five (25) year old patient with +2.00D manifest refraction and +3.50 D cycloplegic refraction can have +3.50D correction via photoablation on the cornea with -0.75D correction (e.g., half of the refraction discrepancy) provided by a corneal inlay (e.g., via ablation of the inlay or lens shape). This combination provides the patient with a total of +2.75D correction. The corneal inlay may be subsequently remove to provide a total of +3.50D correction for a full refractive correction. In the event the patient's hyperopic correction changes over time, the inlay may be photoablated or exchanged.

Lenticular change becomes more apparent typically starting in the forties. The overall refractive index of the lens tends to decrease with aging, and the lens can become thicker, both of which alter the refraction of the lens. An implanted intra-corneal lens can be photoablated or exchanged to fine tune the changing refraction of the lens. Intra-corneal inlays can also be used to provide partial correction or gross correction in combination with corneal photoablation for fine correction, including higher order aberrations, for patients with thin corneas that may not have sufficient residual stromal bed following photoablation. In all of these applications, the system 10, 70, 80 may be used for the photoablative refractive corrections on or in the cornea and one or in the corneal implant.

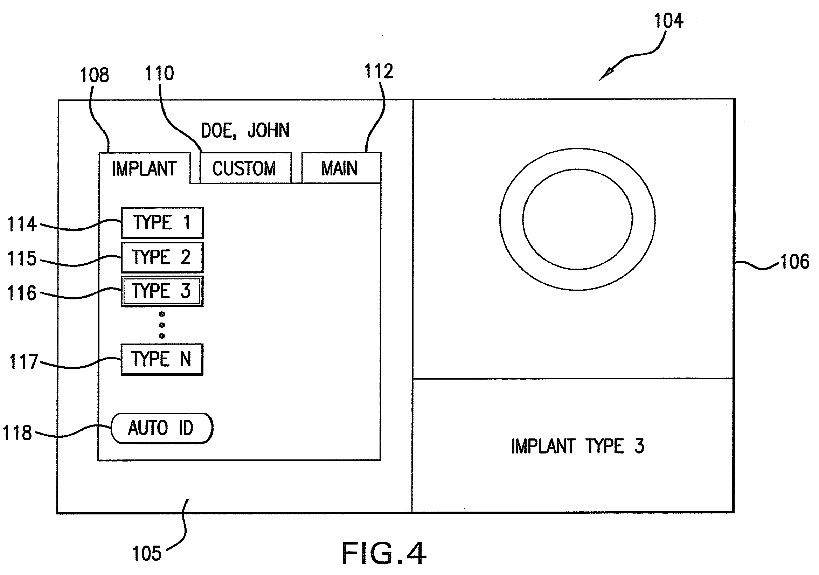

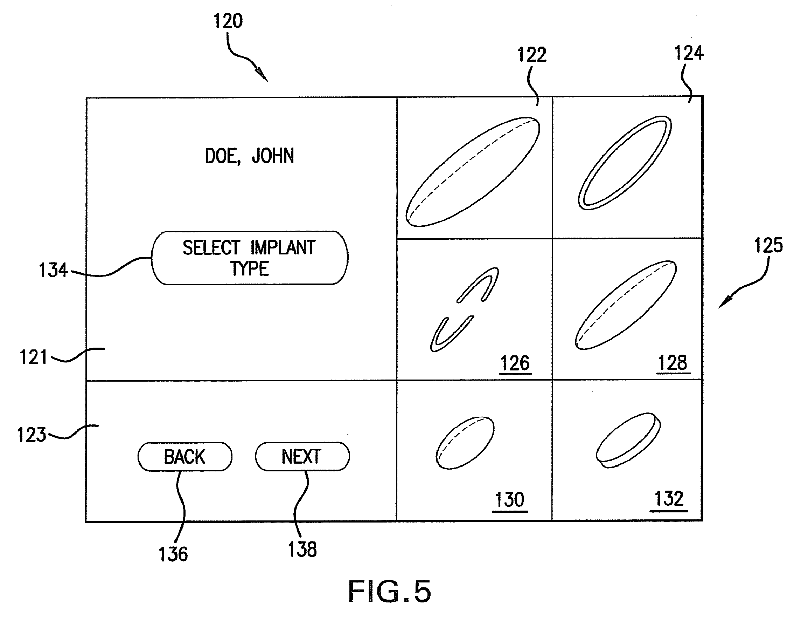

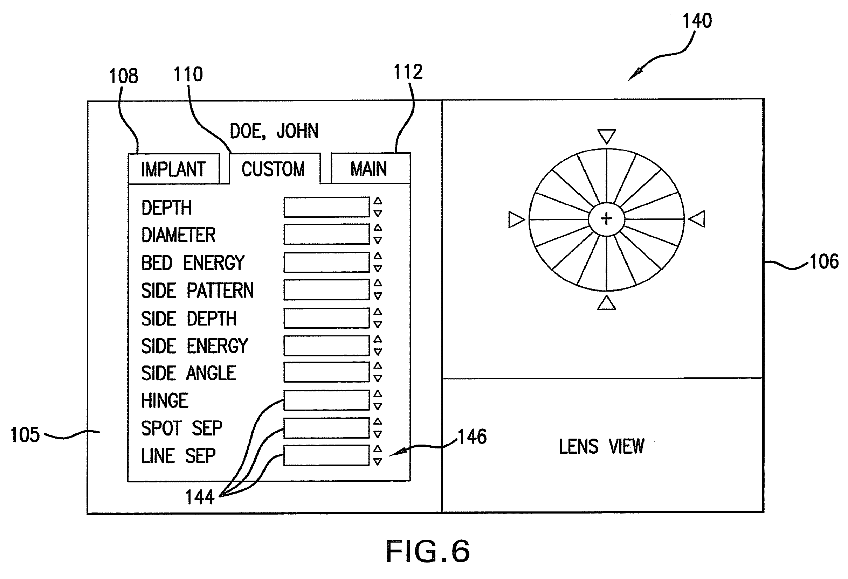

FIG. 4 is an elevational view of a display screen 104 illustrating a graphical user interface in accordance with one embodiment. FIG. 5 is an elevational view of a display screen 120 illustrating a graphical user interface in accordance with another embodiment. FIG. 6 is an elevational view of a display screen 140 illustrating a graphical user interface in accordance with another embodiment. The screens 104, 120, 140 may each be displayed on the user interface 28 (FIG. 1) or 60 (FIG. 2), the display 84 (FIG. 3), or the like and is partitioned into multiple areas. Referring to FIG. 4, the GUI includes, but is not necessarily limited to, a first area 105 of the screen 104 and a second area 106 of the screen 104. The first area 105 presents general system operating parameters and navigation tools, and the second area 106 presents a representation (e.g., a photo image) of a selected corneal implant. In this embodiment, the first area 105 provides tabbed pages 108, 110, 112 (e.g., an IMPLANT tabbed page 108, a CUSTOM tabbed page 110, and a MAIN tabbed page 112) of various system operating parameters for selection, entry of parameter values, modification, or any combination thereof. The type and variety of tabbed pages may vary based on user convenience, ergonomics, other ease-of use considerations, or the like. Additionally, the organization of the graphical user interface may vary. For example, the tabbed pages 108, 110, 112 may be substituted by a toolbar providing the general system operating parameters with drop-down menus for each of the parameters. The screens 104, 120, 140 may also include additional windows, such as for eye images and diagnostics displays (e.g., OCT, wavefront analysis, and the like).

In general, selection of a particular selectable item presented on the display screen 104 may be performed by the user input device 92 (FIG. 3), by a touch-screen coupled with the display 84 (FIG. 3), or the like. The IMPLANT tabbed page 108 has been selected and lists buttons 114, 115, 116, 117 corresponding with the different corneal implant types (e.g., Type 1, Type 2, Type 3, ..., and Type N) available for selection. Selecting one of the other tabbed pages (e.g., the tabbed pages 110 or 112) would display parameters corresponding with the tabbed page. Each of the corneal implant types on the IMPLANT page 108 is represented by a selectable button. For example, a first button 114 represents the Type 1 implant, a second button 115 represents the Type 2 implant, and a third button 116 represents the Type 3 implant, ..., and another button 117 represents the Type N implant. The button 116 indicates that the Type 3 implant has been selected, and a representation (e.g., a photograph or other image) of the Type 3 implant is presented in the second area 106. Additional parameters and navigation tools may also be presented on the display screen 104. For example, an AUTO ID button 118 is presented in the first area 105. When the button 118 is selected, the identification of the corneal implant type may be determined via the read unit 94 (FIG. 3) (e.g., by RFID, bar code scanning, or the like). The incision type may be displayed for confirmation with the selected implant. Additionally, an alert may be provided by the display 84 in the event that the corneal implant type does not match with the particular incision.

Referring to FIG. 5, the GUI includes, but is not necessarily limited to, a first area 121 of the screen 120, a second area 123 of the screen 120, and a third area 125 of the screen 120. In this embodiment, the first area 121 presents a prompt for implant selection, the second area 123 presents navigational tools (e.g., a BACK button 136 for returning to a previously displayed screen and a NEXT button 138 for advancing to a subsequent screen), and the third area 125 presents representations (e.g., images) of a selected corneal implant for user selection. The third area 125 includes, but is not necessarily limited to, a first selectable image 122 representing a corneal implant with a central pin-hole, a second selectable image 124 representing a ring implant, a third selectable image 126 representing a two-piece corneal implant, a fourth selectable image 128 representing a disc or a lens, a fifth selectable image 130 representing a small disc or lens, and a sixth selectable image 132 representing a substantially planar disc or lens. The corneal implants shown in FIG. 5 are merely exemplary, and other types of corneal implants may be substituted for one or more of the selectable images 122, 124, 126, 128, 130, 132 or included as part of a larger collection displayed over this screen 120 and other screens.

Referring to FIG. 6, the GUI includes, but is not necessarily limited to, a first area 141 of the screen 140 and a second area 142 of the screen 140. The first area 141 is similar to the first area 105 shown in FIG. 4 and presents general system operating parameters and navigation tools. The second area 142 provides a view of the patient's eye, such as via the video camera 54 shown in FIG. 2. In the first area 141, the CUSTOM tabbed page 110 has been selected and lists various incision parameters (e.g., depth, diameter, bed energy, side pattern, side depth, side energy, side angle, hinge, spot separation, and line separation), which are exemplary and not inclusive. Each of these parameters is associated with an entry block 144 that displays a current parameter setting. One or more of these parameter settings may be modified using incremental arrows 146 associated with each block 144. For example, based on the selected or identified corneal implant type, the entry blocks 144 display parameter settings (e.g., default values stored in the catalog 98 shown in FIG. 3) associated with incision procedure corresponding with the selected or identified corneal implant type. These parameter settings may be increased by selecting an upward arrow and decreased by selecting a downward arrow.

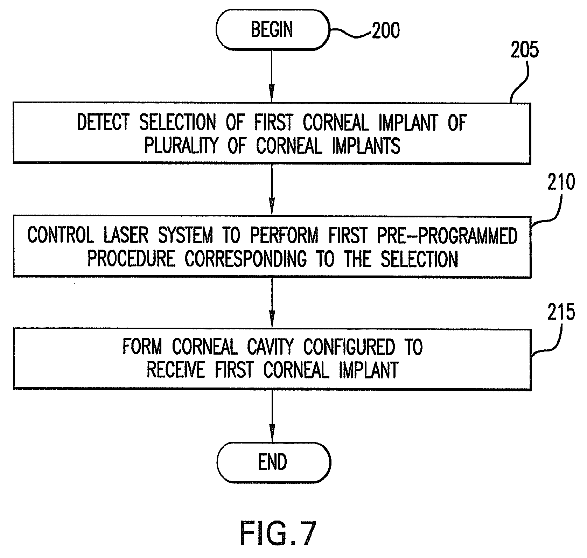

FIG. 7 is a flow diagram of a method 200 for ophthalmic surgery given as an example not part of the invention. A selection of a first corneal implant of a plurality of corneal implants is detected, as indicated at step 205. In one embodiment, a plurality of items representing a plurality of corneal implants is displayed. In this embodiment, selection of a first item from the plurality of items is detected, where the first item represents the first corneal implant. Referring to FIGS. 1-4 and 7, the items may be the selectable buttons 114, 115, 116, 117 provided on the screen 104, or the selectable images 122, 124, 126, 128, 130, 132 provided on the screen 120, for example. In another embodiment, a first identification of a plurality of identifications is detected, where the first identification represents the first corneal implant. For example, the read unit 94 detects the identification associated with a desired corneal implant by scanning a bar code on the packaging for the desired corneal implant, interrogating an RFID tag included in the packaging for the desired corneal implant, or the like.

A laser system is controlled to perform a first pre-programmed procedure corresponding to the selection, as indicated at step 210. For example, the CPU 82 selects and retrieves the corresponding incision procedure from the incision catalog 98 corresponding with the selected corneal implant. The CPU 82 controls the laser assembly 86 to perform the corresponding incision procedure. A corneal cavity configured to receive the first corneal implant is formed, as indicated at step 215, and the corneal implant may be implanted in the corneal cavity.

Thus, systems, graphical user interfaces, and methods for ophthalmic surgery are disclosed that operate with and manage multiple corneal implant types, establish a corneal pocket suitable for accommodating a selected corneal implant, and/or automatically select a pre-programmed incision procedure based on a corneal implant desired for implantation. Examples of additional refractive eye surgery applications for the systems 10, 70, and 80 include, but are not necessarily limited to, photorefractive keratectomy (PRK), LASIK, laser assisted sub-epithelium keratomileusis (LASEK), corneal transplant, intraocular lens or the like. Although the systems 10, 70, and 80 are capable of forming a corneal pocket for receiving a corneal implant, the systems 10, 70, and 80 may also be used to form cavities within various regions of the eye, such as for intraocular lens procedures and the like.

The description of the invention and its applications as set forth herein is illustrative and is not intended to limit the scope of the invention. Variations and modifications of the embodiments disclosed herein are possible, and practical alternatives to and equivalents of the various elements of the embodiments would be understood to those of ordinary skill in the art upon study of this patent document. These and other variations and modifications of the embodiments disclosed herein may be made without departing from the scope of the invention.

## Claims

1. A system (80) for performing ophthalmic surgery on a cornea, the system comprising:
a display (84) operable to present a plurality of images representing a plurality of corneal implants;
a laser assembly (86) operable to output a pulsed laser beam; and
a control unit (82) coupled to the laser assembly and the display, the control unit configured to direct the laser assembly in response to a selected image of the plurality of images to produce a cavity in the cornea via the pulsed laser beam, the cavity configured to receive a first corneal implant of the plurality of corneal implants corresponding to the selected image, in particular wherein the first corneal implant has a shape, and wherein the cavity conforms with the shape, **characterized by**
a memory (88) coupled to the control unit, the memory comprising a database of a plurality of procedures; wherein the database preferably comprises a set of predetermined instructions to form a different cavity in the cornea; and wherein the control unit is further configured to:
select a first procedure from the database in response to the selected image; and
direct the laser assembly in accordance with the first procedure to produce the cavity in the cornea.

2. A system of claim 1, wherein the control unit is further configured to detect a selection of the selected image of the plurality of images, the selected image representing a first corneal implant of the plurality of corneal implants.

3. A system of claim 2, wherein the display comprises a touch-sensitive screen, and wherein the control unit is further configured to detect a user contact on the touch-sensitive screen corresponding with the selection of the selected image of the plurality of images.

4. A system of claim 1, further comprising an input device (92) coupled to the control unit and the display, the input device configured to indicate to the control unit a selection of the selected image of the plurality of images.

5. The system of claim 1, wherein the control unit is further configured for one of a group consisting of:
directing the laser assembly to photoablate the first corneal implant after the first corneal implant is received by the cavity; and
directing the laser assembly to photoablate a desired region of the cornea and thereby produce a refractive correction in combination with the first corneal implant.

6. The system of claim 1, wherein the control unit is further configured to direct the laser assembly to photoablate the corneal implant after the corneal implant is received by the cavity, and/or
wherein the control unit is further configured to direct the laser assembly to photoablate a desired region of the cornea and thereby produce a refractive correction in combination with the corneal implant.

## Patentansprüche

1. System (80) zum Durchführen einer Augenoperation an einer Hornhaut, wobei das System umfasst:
eine Anzeige (84), die so ausgelegt ist, dass sie eine Vielzahl von Bildern präsentiert, welche eine Vielzahl von Hornhautimplantaten darstellen;
eine Laseranordnung (86), die so ausgelegt ist, dass sie einen gepulsten Laserstrahl ausgibt; und
eine Steuereinheit (82), die mit der Laseranordnung und der Anzeige gekoppelt ist, wobei die Steuereinheit so ausgeführt ist, dass sie die Laseranordnung in Reaktion auf ein ausgewähltes Bild der Vielzahl von Bildern führt, um mittels des gepulsten Laserstrahls eine Vertiefung in der Hornhaut herzustellen, wobei die Vertiefung so ausgeführt ist, dass sie ein erstes Hornhautimplantat der Vielzahl von Hornhautimplantaten aufnimmt, das dem ausgewählten Bild entspricht, wobei insbesondere das erste Hornhautimplantat eine Gestalt aufweist und wobei die Vertiefung mit der Gestalt übereinstimmt, **gekennzeichnet durch**
einen Speicher (88), der mit der Steuereinheit gekoppelt ist, wobei der Speicher eine Datenbank einer Vielzahl von Vorgängen umfasst; wobei die Datenbank vorzugsweise einen Satz von vorbestimmten Anweisungen zum Ausbilden einer unterschiedlichen Vertiefung in der Hornhaut umfasst; und wobei die Steuereinheit ferner so ausgeführt ist, dass sie:
in Reaktion auf das ausgewählte Bild einen ersten Vorgang aus der Datenbank auswählt; und
die Laseranordnung entsprechend dem ersten Vorgang führt, um die Vertiefung in der Hornhaut herzustellen.

2. System nach Anspruch 1, wobei die Steuereinheit ferner so ausgeführt ist, dass sie eine Auswahl des ausgewählten Bilds der Vielzahl von Bildern detektiert, wobei das ausgewählte Bild ein erstes Hornhautimplantat der Vielzahl von Hornhautimplantaten darstellt.

3. System nach Anspruch 2, wobei die Anzeige einen Sensorbildschirm umfasst und wobei die Steuereinheit ferner so ausgeführt ist, dass sie einen Nutzerkontakt mit dem Sensorbildschirm detektiert, der der Auswahl des ausgewählten Bilds der Vielzahl von Bildern entspricht.

4. System nach Anspruch 1, das ferner eine Eingabevorrichtung (92) umfasst, die mit der Steuereinheit und der Anzeige gekoppelt ist, wobei die Eingabevorrichtung so ausgeführt ist, dass sie der Steuereinheit eine Auswahl des ausgewählten Bilds der Vielzahl von Bildern anzeigt.

5. System nach Anspruch 1, wobei die Steuereinheit ferner ausgeführt ist für eines einer Gruppe bestehend aus:
Führen der Laseranordnung für eine Fotoablation des ersten Hornhautimplantats, nachdem das erste Hornhautimplantat in der Vertiefung aufgenommen worden ist; und
Führen der Laseranordnung für eine Fotoablation einer gewünschten Region der Hornhaut und dadurch Erstellen einer refraktiven Korrektur in Kombination mit dem ersten Hornhautimplantat.

6. System nach Anspruch 1, wobei die Steuereinheit ferner so ausgeführt ist, dass sie die Laseranordnung für eine Fotoablation des Hornhautimplantats führt, nachdem das Hornhautimplantat in der Vertiefung aufgenommen worden ist, und/oder
wobei die Steuereinheit ferner so ausgeführt ist, dass sie die Laseranordnung für eine Fotoablation einer gewünschten Region der Hornhaut führt und dadurch eine refraktive Korrektur in Kombination mit dem Hornhautimplantat erstellt.

## Revendications

1. Système (80) destiné à effectuer une chirurgie ophtalmique sur une cornée, le système comprenant :
un affichage (84) utilisable pour présenter une pluralité d'images représentant une pluralité d'implants cornéens ;
un ensemble laser (86) utilisable pour émettre un faisceau laser pulsé ; et
une unité de commande (82) couplée à l'ensemble laser et à l'affichage, l'unité de commande étant configurée pour diriger l'ensemble laser en réponse à une image sélectionnée parmi la pluralité d'images pour produire une cavité dans la cornée par l'intermédiaire du faisceau laser pulsé, la cavité configurée pour recevoir un premier implant cornéen de la pluralité d'implants cornéens correspondant à l'image sélectionnée, en particulier où le premier implant cornéen a une forme, et où la cavité correspond à la forme, **caractérisé par** :
une mémoire (88) couplée à l'unité de commande, la mémoire comprenant une base de données d'une pluralité de procédures ; où la base de données comprend de préférence un ensemble d'instructions prédéterminées pour former une cavité différente dans la cornée ; et
où l'unité de commande est en outre configurée pour :
sélectionner une première procédure dans la base de données en réponse à l'image sélectionnée ; et
diriger l'ensemble laser conformément à la première procédure pour produire la cavité dans la cornée.

2. Système selon la revendication 1, dans lequel l'unité de commande est en outre configurée pour détecter une sélection de l'image sélectionnée de la pluralité d'images, l'image sélectionnée représentant un premier implant cornéen de la pluralité d'implants cornéens.

3. Système selon la revendication 2, dans lequel l'affichage comprend un écran tactile, et dans lequel l'unité de commande est en outre configurée pour détecter un contact d'utilisateur sur l'écran tactile correspondant à la sélection de l'image sélectionnée de la pluralité d'images.

4. Système selon la revendication 1, comprenant en outre un dispositif d'entrée (92) couplé à l'unité de commande et à l'affichage, le dispositif d'entrée étant configuré pour indiquer à l'unité de commande une sélection de l'image sélectionnée de la pluralité d'images.

5. Système selon la revendication 1, dans lequel l'unité de commande est en outre configurée pour exécuter une étape parmi celles du groupe suivant :
diriger l'ensemble laser pour procéder à une photoablation du premier implant cornéen après que le premier implant cornéen a été reçu par la cavité ; et
diriger l'ensemble laser pour procéder à une photoablation d'une région souhaitée de la cornée et ainsi produire une correction réfractive en combinaison avec le premier implant cornéen.

6. Système selon la revendication 1, dans lequel l'unité de commande est en outre configurée pour diriger l'ensemble laser pour procéder à une photoablation de l'implant cornéen après que l'implant cornéen a été reçu par la cavité ; et/ou dans lequel l'unité de commande est en outre configurée pour diriger l'ensemble laser pour procéder à une photoablation d'une région souhaitée de la cornée et ainsi produire une correction réfractive en combinaison avec le premier implant cornéen.
